(19) Europäisches Patentamt
European Patent Office
Office européen des brevets

(11) Numéro de publication: **0 324 111 B1**

(12) **FASCICULE DE BREVET EUROPEEN**

(45) Date de publication de fascicule du brevet: **09.09.92**

(51) Int. Cl.⁵: **C07C 45/54**, C07C 47/32, C07C 47/347

(21) Numéro de dépôt: **88120576.9**

(22) Date de dépôt: **09.12.88**

(54) **Procédé pour la préparation d'aldéhydes cycloaliphatiques.**

(30) Priorité: **14.01.88 CH 133/88**

(43) Date de publication de la demande: **19.07.89 Bulletin 89/29**

(45) Mention de la délivrance du brevet: **09.09.92 Bulletin 92/37**

(84) Etats contractants désignés: **CH DE FR GB IT LI NL**

(56) Documents cités:
**EP-A- 0 255 904**
**DE-B- 1 045 393**

(73) Titulaire: **FIRMENICH SA**
**1, route des Jeunes Case Postale 239**
**CH-1211 Genève 8(CH)**

(72) Inventeur: **Simmons, Dana P.**
**1105 Edinburgh Court**
**Jamestown, NC 27282(US)**
Inventeur: **Chapuis, Christian**
**12, chemin du Champ d'Anier**
**CH-1209 Petit-Saconnex(CH)**

(74) Mandataire: **Salvadori, Giuseppe, Dr.**
**c/o Firmenich S.A. Case Postale 239**
**CH-1211 Genève 8(CH)**

**EP 0 324 111 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention a trait au domaine de la synthèse organique, tout particulièrement elle concerne un procédé nouveau pour la préparation d'aldéhydes cycloaliphatiques dont l'intérêt réside dans le fait qu'ils constituent des intermédiaires utiles pour la préparation de produits destinés à l'industrie des arômes et de la parfumerie, ainsi qu'à la préparation de composés biologiquement actifs notamment de la série des drimanes.

La demande de brevet européen n° 255'904, déposée par la demanderesse le 25 juillet 1987, et publiée le 17.02.88 décrit un procédé pour la préparation de 2,2,6-triméthyl-cyclohexane-carboxaldéhyde, lequel procédé est caractérisé par la cyclisation au moyen d'un agent de cyclisation acide d'un énol-ester de formule

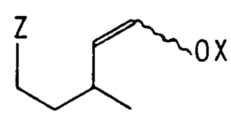

dans laquelle la ligne ondulée définit une liaison C-O de configuration cis ou trans, X représente un groupe acyle ou $P(0)(0R)_2$, R étant un radical monovalent alkyle inférieur ou aryle, et Z définit un groupe monovalent de formule

a. $CH = C(CH_3)_2$,
b. $CH_2 - C(OH)(CH_3)_2$, ou
c. $CH_2 - C(CH_3) = CH_2$

Nous avons maintenant établi que le principe sur lequel repose ledit procédé pouvait s'appliquer de manière tout à fait analogue à la synthèse d'autres aldéhydes cycloaliphatiques dont la structure est définie à l'aide de la formule générale suivante

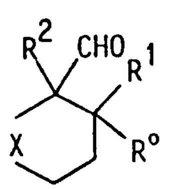

(I)

dans laquelle chacun des symboles $R°$, $R^1$ et $R^2$ représentent, pris isolément, un atome d'hydrogène ou un alkyle inférieur de $C_1$ à $C_6$ et X sert à définir un groupe de formule

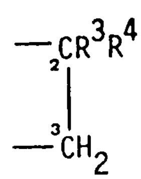

,

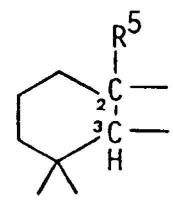

pour lesquelles $R^3$ et $R^4$ représentent, pris isolément, un radical alkyle inférieur de $C_1$ à $C_3$ et $R^5$ représente un reste alkyle inférieur ou l'hydrogène, étant entendu que $R^1$, $R^3$ et $R^4$ ne peuvent représenter simultanément un radical méthyle lorsque $R°$ et $R^2$ représentent chacun un atome d'hydrogène.

La présente invention a donc pour objet un procédé pour la préparation des aldéhydes de formule (I) caractérisé en ce qu'on cyclise au moyen d'un agent de cyclisation acide un énol-ester de formule

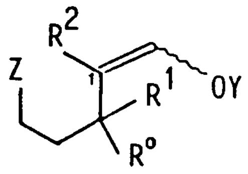

(II)

dans laquelle la ligne ondulée définit une liaison C-O de configuration cis ou trans, Y représente un groupe acyle ou $P(0)(0R)_2$, R étant un radical monovalent alkyle inférieur ou aryle, et Z définit un groupe de formule

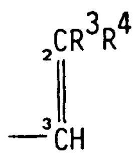 , 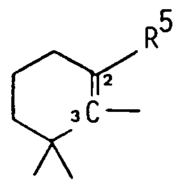 ou 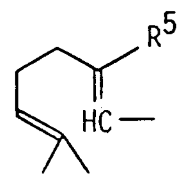

pour lesquelles $R^3$, $R^4$ et $R^5$ ont le sens indiqué plus haut.

Ainsi le radical R peut représenter un groupe alkyle $C_1$-$C_6$, par exemple méthyle, éthyle, propyle ou isopropyle ou un groupe phényle et Y représente un groupe acyle du type R'CO, R' étant de préférence un radical alkyle inférieur de $C_1$ à $C_4$.

Ces esters, tout comme les autres esters dialkylphosphates définis par la formule (II), peuvent être préparés à partir des aldéhydes de formule

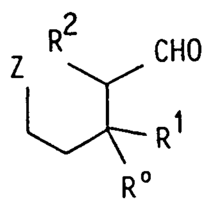

(III)

dans laquelle les symboles $R^{\circ}$, $R^1$, $R^2$ et Z ont le sens indiqué plus haut, selon un procédé analogue à ceux décrits dans la littérature [voir par exemple : J. Am. Chem. Soc., 72, 2617 (1950)]. Par exemple on peut effectuer cette préparation à l'aide d'un traitement des aldéhydes de formules (III) avec l'anhydride acétique en présence d'un agent basique, telle une amine tertiaire comme la triéthylamine ou en présence d'un carbonate alcalin, par exemple le carbonate de sodium.

A titre d'agents de cyclisation acides, on peut utiliser les acides protiques, organiques ou minéraux ou les acides de type de Lewis. Parmi les agents préférentiels, il convient de citer les acides sulfurique, phosphorique, polyphosphorique, méthanesulfonique, acétique, trifluoroacétique ou encore, parmi les acides de Lewis, le tétrachlorure d'étain, le tétrachlorure de titanium ou le trifluorure de bore par exemple.

La température à laquelle on effectue la réaction de cyclisation n'est pas critique et peut varier dans une gamme de valeurs assez étendue. Elle est choisie généralement en fonction de l'agent acide utilisé. C'est ainsi que de bons rendements en produit final ont été obtenus à 0°C en utilisant, comme agent acide, l'acide sulfurique. De très bons rendements ont également été obtenus par traitement à 100°C des énol-esters avec l'acide phosphorique, par exemple l'acide phosphorique à 85 %, ou l'acide polyphosphorique. Des températures supérieures ou inférieures aux valeurs indiquées peuvent également être employées.

Des modes particuliers d'exécution du procédé de l'invention seront indiqués dans les exemples spécifiques de préparation donnés ci-après. Le procédé de l'invention présente des avantages certains vis-à-vis des procédés connus de l'art antérieur pour la préparation des aldéhydes du type de ceux définis par la formule (I). Il se distingue par une plus grande simplicité des opérations requises, ce qui se traduit par une plus grande économie. A cela s'ajoute un avantage majeur : grace au procédé de l'invention, il est en effet désormais possible de préparer des aldéhydes optiquement actifs dans la mesure où la cyclisation qui

caractérise le procédé s'accompagne de manière surprenante de la rétention de la configuration propre à l'énol-ester de départ.

L'invention est illustrée à l'aide des exemples suivants dans lesquels les températures sont indiquées en degrés centigrades et les abréviations ont le sens usuel dans l'art.

## Exemples

### Méthode générale

Une solution concentrée dans le toluène de l'énol-ester de départ est mélangée à de l'acide phosphorique à 85 % (3,5 mole équivalents) et à un volume identique de toluène. Le mélange est ensuite chauffé à 100° pendant 2 heures, puis, après refroidissement à température ambiante, il est versé dans de l'eau et extrait au toluène. Les extraits organiques combinés sont ensuite lavés avec une solution aqueuse saturée de bicarbonate de sodium et une solution aqueuse saturée de chlorure de sodium, puis ils sont séchés sur du sulfate de sodium anhydre et concentrés. Les aldéhydes désirés sont enfin obtenus par une distillation fractionnée.

En procédant comme indiqué ci-dessus on a préparé les aldéhydes suivants :

1. 2,2,6,6-tétraméthyl-1-cyclohexanecarbaldéhyde :

| | |
|---|---|
| Eb. | 45°/6,6 × $10^2$ Pa ; rend. 75 %; |
| IR: | 1710, 2710 $cm^{-1}$; |
| RMN | (360 MHz) : 0,94 ; 1,17(2s,12H) ; 9,91(d,J=5Hz,1H) delta ppm; |
| SM : | $M^+$ = 168(2) ; m/e : 153(3), 135(11), 125(18), 112(5), 109(21), 95(18), 85(100), 72(19), 69-(63), 55(40), 41(32). |

2. 1 alpha, 2,2,6 alpha-tétraméthyl-1-cyclohexanecarbaldéhyde :

| | |
|---|---|
| Eb. | 86-7°/20 × $10^2$ Pa ; rend. 27 %; |
| IR : | 900, 1145, 1370, 1450, 1700, 3000 $cm^{-1}$; |
| RMN | (360 MHz) : 0,73(d,J=7,3H) ; 0,84(s,3H) ; 0,95(s,3H) ; 1,12(s,3H) ; 2,35(m,1H) ; 9,62(s,1H) delta ppm; |
| SM : | $M^+$ = 168(5) ; m/e : 153(9), 135(11), 125(7), 109(24), 83(72), 69(83), 57(85), 41(100). |

3. 2,6 alpha-diméthyl-2 alpha-éthyl-1 bêta-cyclohexanecarbaldéhyde :

| | |
|---|---|
| Eb. | 89-92°/21,3 x $10^2$ Pa ; rend. 42 %; |

4. 2,6,6 triméthyl-2-butyl-1-cyclohexanecarbaldéhyde :

| | |
|---|---|
| rend. | 30 %; |
| IR: | 1710, 2710 $cm^{-1}$; |
| RMN | (360 MHz) : 0,88(t,J=13,3H) ; 0,93(s,3H) ; 1,17-1,18(2s,6H); 9,89 ; 9,92(2d,J=5,1H) delta ppm; |
| SM | : $M^+$ = 210(1) ; m/e : 192(2), 177(8), 163(17), 149(5), 135(13), 125(22), 109(51), 95(42), 85-(100), 69(83), 55(59), 41(44). |

5. 1 alpha-formyl-2 bêta,5,5,8a alpha-tétraméthyl-4a bêta-décahydronaphtalène:

| | |
|---|---|
| rend. | 43 % ; Eb. 100-120°/1,2 x $10^2$ Pa |
| IR : | 2925, 1720, 1455, 1387, 1370, 1193, 1170, 1035, 987 $cm^{-1}$; |
| RMN | (360 MHz) : 0,78(d,J=7,3H) ; 0,84(s,3H) ; 0,86(s,3H) ; 1,09(s,3H) ; 1,90(dq,J=13,3,1H) ; 2,08(m,1H) ; 9,69(d,J=4,1H) delta ppm |
| RMN | ($^{13}C$) : 15,98(q) ; 18,40(t) ; 20,66(q) ; 21,67(t) ; 21,88(q) ; 27,63(d) ; 33,17(s) ; 33,52(q) ; 35,61(t) ; 38,50(s) ; 40,32(t) ; 41,96(t) ; 54,34(d) ; 70,43(d) ; 207,65(d) delta ppm; |
| SM : | $M^+$ = 222(9); m/e : 207(11), 189(13), 138(67), 123(100), 109(62), 95(68), 84(81), 69(76), 55-(59), 43(96). |

Ce dernier composé a été obtenu par cyclisation de l'énol-acétate correspondant à l'aide de $SnCl_4$ dans le dichlorométhane.

6. 1 alpha-formyl-5,5, 8a alpha-triméthyl-4a bêta-décahydronaphtalène:

| | |
|---|---|
| rend. | 36 % ; Eb. 140°/1,2 x $10^2$ Pa |
| IR : | 2920, 2870, 2710, 1715, 1440, 1383, 1360, 1167, 950 $cm^{-1}$; |
| RMN | (360 MHz) : 0,83(s,3H) ; 0,86(s,3H) ; 1,01(s,3H) ; 1,15-1,73(m,11H) ; 1,80-2,02(m,3H) ; 9,62-(d,J=2,1H) delta ppm; |
| RMN | ($^{13}C$) : 15,51(q) ; 18,49(t) ; 21,48(t) ; 21,60(q) ; 22,07(t) ; 26,02(t) ; 33,28(s) ; 33,44(q) ; 54,63-(d) ; 63,39(d) ; 206,24(d) delta ppm. |
| SM : | $M^+$ = 208(6) ; m/e : 138(22), 123(81), 109(45), 95(86), 81(92), 69(100), 55(52), 41(30). |

La méthode générale pour la préparation des énol-esters de départ est illustrée par l'exemple suivant :

0,063 M de 3,3,7-triméthyl-oct-6-ène-1-al ont été ajoutées sous agitation à un mélange constitué par 0,06 M de triéthylamine, 0,126 M d'anhydride acétique et 1,1 g d'acétate de potassium préchauffé à 80°. Le mélange réactionnel a été maintenu à reflux pendant 7 h environ.

Après refroidissement à température ambiante, on y a ajouté du toluène et de l'eau et les deux phases ont été séparées. L'acétate de 3,3,7-triméthyl-1,6-octadiényle a été ensuite obtenu sous forme de solution dans le toluène et isolé enfin par évaporation sous pression réduite.

De manière analogue on a obtenu les autres énol-esters servant comme produits de départ dans le procédé de l'invention.

Le tableau suivant indique certains des produits obtenus ainsi que les produits de départ utilisés.

Tableau

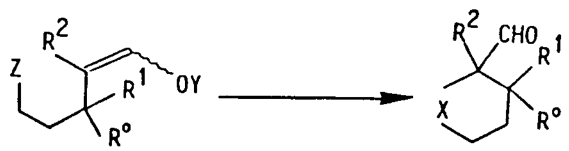

| | R° | $R^1$ | $R^2$ | X | $R^3$ | $R^4$ | $R^5$ | Z | Y |
|---|---|---|---|---|---|---|---|---|---|
| 1. | $CH_3$ | $CH_3$ | H | $CR^3R^4$<br>$\vert$<br>$CH_2$ | $CH_3$ | $CH_3$ | - | $C(CH_3)_2$<br>$\Vert$<br>CH | $CH_3CO$ |
| 2. | H | $CH_3$ | $CH_3$ | $CR^3R^4$<br>$\vert$<br>$CH_2$ | $CH_3$ | $CH_3$ | - | $C(CH_3)_2$<br>$\Vert$<br>CH | $CH_3CO$ |
| 3. | H | $CH_3$ | H | $CR^3R^4$<br>$\vert$<br>$CH_2$ | $C_2H_5$ | $CH_3$ | - | $CCH_3(C_2H_5)$<br>$\Vert$<br>CH | $CH_3CO$ |
| 4. | $CH_3$ | $C_4H_9$ | H | $CR^3R^4$<br>$\vert$<br>$CH_2$ | $CH_3$ | $CH_3$ | - | $C(CH_3)_2$<br>$\Vert$<br>CH | $CH_3CO$ |
| 5. | H | $CH_3$ | H | 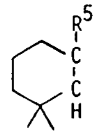 | - | - | $CH_3$ | 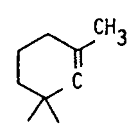 | $CH_3CO$ |
| 6. | H | H | H | 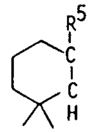 | - | - | H | 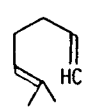 | $CH_3CO$ |

**Revendications**

**1.** Procédé pour la préparation d'aldéhydes cycloaliphatiques de formule

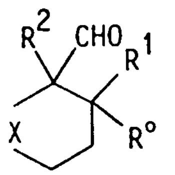

(I)

dans laquelle R°, $R^1$ et $R^2$ représentent indépendamment un atome d'hydrogène ou un alkyle inférieur de $C_1$ à $C_6$ et X sert à définir un groupe de formule

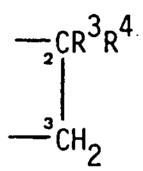

ou

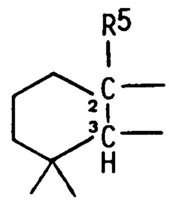

pour lesquelles $R^3$ et $R^4$ représentent indépendamment un radical alkyle inférieur de $C_1$ à $C_3$ et $R^5$ représente un reste alkyle inférieur ou l'hydrogène, étant entendu que $R^1$, $R^3$ et $R^4$ ne peuvent représenter simultanément un radical méthyle lorsque R° et $R^2$ représentent chacun un atome d'hydrogène, caractérisé en ce qu'on cyclise au moyen d'un agent de cyclisation acide un énol-ester de formule

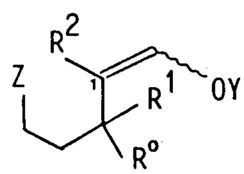

(II)

dans laquelle la ligne ondulée définit une liaison C-0 de configuration cis ou trans,Y représente un groupe acyle ou $P(0)(0R)_2$, R étant un radical monovalent alkyle inférieure ou aryle, et Z définit un groupe de formule

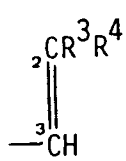

,

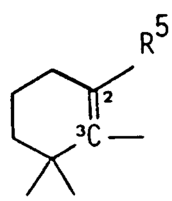

ou

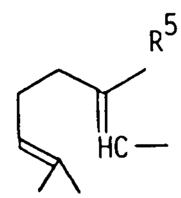

pour lesquelles $R^3$, $R^4$ et $R^5$ ont le sens indiqué plus haut.

**2.** Procédé selon la revendication 1 caractérisé en ce qu'on utilise à titre d'agent de cyclisation acide un acide protonique organique ou minéral ou un acide du type de Lewis.

**3.** Procédé selon la revendication 2 caractérisé en ce qu'on utilise à titre d'agent de cyclisation l'acide acétique, l'acide trifluoroacétique, l'acide phosphorique, l'acide méthanesulfonique, le trifluorure de bore, le tétrachlorure de titanium ou le tétrachlorure d'étain.

**4.** Procédé selon la revendication 3 caractérisé en ce qu'on effectue la cyclisation à une température comprise entre environ 0° et environ 100° C.

**5.** Procédé selon l'une quelconque des revendications précédentes caractérisé en ce qu'on utilise comme

produit de départ un énol-ester optiquement actif de formule

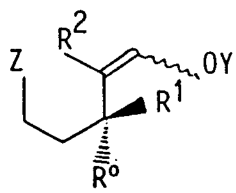

ou

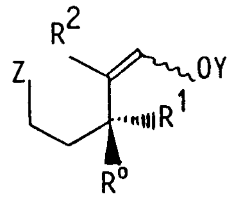

dans lesquelles Z, la ligne ondulée et les substituants R°, $R^1$ et $R^2$ ainsi que Y ont le sens indiqué à la revendication 1 et que l'on obtient un aldéhyde cycloaliphatique isomériquement équivalent.

## Claims

**1.** A process for the preparation of cycloaliphatic aldehydes of formula

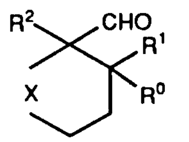

(I)

wherein $R^0$, $R^1$ and $R^2$ represent independently a hydrogen atom or a lower alkyl from $C_1$ to $C_6$ and X stands for a group of formula

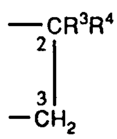

or

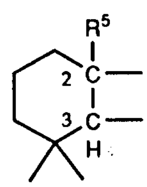

wherein $R^3$ and $R^4$ represent independently a lower alkyl radical from $C_1$ to $C_3$ and $R^5$ represents a lower alkyl radical or hydrogen, provided that $R^1$, $R^3$ and $R^4$ do not represent simultaneously a methyl radical whenever $R^0$ and $R^2$ represent each a hydrogen atom, characterized in that an enol ester of formula

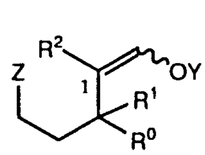

(II)

wherein the wavy line stands for a C-O bond of cis or trans configuration, Y represents an acyl group or $P(O)(OR)_2$, R being a monovalent lower alkyl radical or an aryl, and Z defines a group of formula

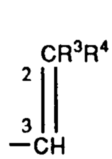

,

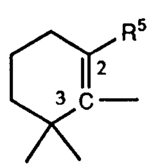

or

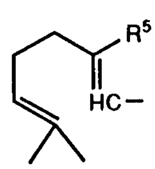

wherein $R^3$, $R^4$ and $R^5$ have the meaning indicated above, is cyclised by means of an acidic cyclisation

agent.

2. A process according to claim 1, characterized in that a protic organic or mineral acid or a Lewis type acid is used as the acidic cyclisation agent.

3. A process according to claim 2, characterized in that acetic acid, trifluoroacetic acid, phosphoric acid, methanesulphonic acid, boron trifluoride, titanium tetrachloride or tin tetrachloride is used as the acidic cyclisation agent.

4. A process according to claim 3, characterized in that the cyclisation is carried out at a temperature comprised between about 0° and about 100°C.

5. A process according to any one of the preceding claims, characterized in that an optically active enol ester of formula

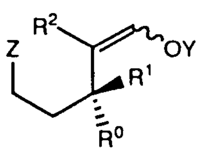

or

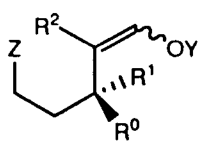

wherein Z, the wavy line and the substituents $R^0$, $R^1$ and $R^2$, as well as Y, have the meaning indicated in claim 1, is used as starting product and in that an isomerically equivalent cycloaliphatic aldehyde is obtained.

## Patentansprüche

1. Verfahren zur Herstellung von cycloaliphatischen Aldehyden der Formel

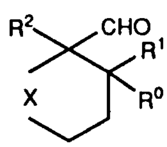

(I)

worin $R^0$, $R^1$ und $R^2$ unabhängig ein Wasserstoffatom oder einen niedrigen $C_1$ bis $C_6$ Alkylrest bedeuten, und X eine Gruppe der Formel

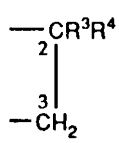

oder

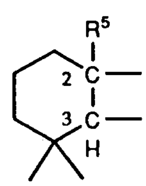

in welcher $R^3$ und $R^4$ unabhängig einen niedrigen $C_1$ bis $C_3$ Alkylrest und $R^5$ einen niedriegen Alkylrest oder ein Wasserstoffatom bedeuten, mit der Einschränkung, daß $R^1$, $R^3$ und $R^4$ nicht gleichzeitig ein Methylrest darstellen können, wenn $R^0$ und $R^2$ ein Wasserstoffatom bedeuten, dadurch gekennzeichnet, daß man mittels eines sauren Cyclisierungsmittels einen Enolester de Formel,

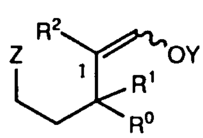

(II)

in welcher die wellige Linie eine cis oder trans C-O Bindung darstellt, Y eine Acyl- oder $P(O)(OR)_2$-Gruppe bedeutet, worin R für einen einwertigen niedrigen Alkyl- oder einen Arylrest steht, und Z eine Gruppe der Formel

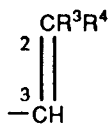
,
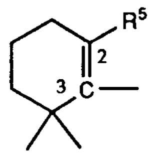
oder
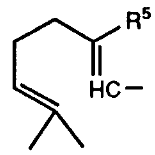

in welchen $R^3$, $R^4$ und $R^5$ die oben angegebene Bedeutung haben, cyclisiert.

**2.** Verfahren gemäß Anspruch 1, dadurch gekennzeichnet, daß man als saures Cyclisierungsmittel eine organische Protonensäure, Mineralsäure oder eine Lewissäure verwendet.

**3.** Verfahren gemäß Anspruch 2, dadurch gekennzeichnet, daß man als saures Cyclisierungsmittel Essigsäure, Trifluoroessigsäure, Phosphorsäure, Methansulfonsäure, Bortrifluorid oder Titantetrachlorid verwendet.

**4.** Verfahren gemäß Anspruch 3, dadurch gekennzeichnet, daß die Cyclisierungsreaktion bei einer Temperatur von ungefähr 0° bis ungefähr 100°C durchgeführt wird.

**5.** Verfahren gemäß einem der vorhergehenden Ansprüche, dadurch gekennzeichnet, daß man als Ausgangsprodukt einen optisch aktiven Enolester der Formel

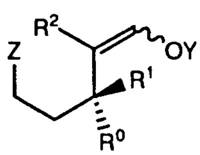
oder
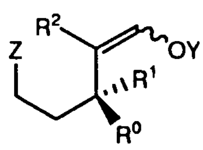

verwendet, in welcher Z, die wellige Linie und die Substituenten $R^0$, $R^1$ und $R^2$, so wie Y, die im Anspruch 1 angegebene Bedeutung haben, und daß man einen cycloaliphatischen Aldehyd mit gleicher Isomerie erhält.